# EUROPÄISCHE PATENTANMELDUNG

(11) **EP 0 712 807 A2**
(43) Veröffentlichungstag der Anmeldung: **22.05.1996**
(21) Anmeldenummer: 95116232.0
(22) Anmeldetag: 14.10.1995
(51) Int. Cl.: C02F 1/48, C02F 1/00, A61L 2/02, A61L 2/18

(54) **Verfahren und Vorrichtung zur Behandlung von Mikroorganismen**

(30) Priorität: 18.11.1994 AT 2145/94
(71) Anmelder: INNUTEC, Innovative Umwelt Technologie Gesellschaft m.b.H., A-6373 Jochberg (DE)
(72) Erfinder: Felsch, Horst, Dipl.-Ing. Dr., A-6391 Fieberbrunn (AT); Grander, Johann, A-6373 Jochberg (AT)
(74) Vertreter: Grabherr-Puchberger, Claudia

(57) **Zusammenfassung**

Microorganismen bzw. ein Microorganismen enthaltendes Medium werden dadurch behandelt, daß ein in seiner elektromagnetischen Struktur durch Änderung der magnetischen Kernresonanzeigenschaften und durch Ausbildung von supermolekularen Komplexen zwischen den Molekülen in seinem Schwingungszustand geändertes Wasser oder Medium indirekt auf das die Mikroorganismen enthaltende Medium einwirkt oder diesem direkt zugesetzt wird.
Damit können Microorganismen nebenwirkungsfrei beeinflußt werden, so daß der Einsatz von Desinfektionsmitteln, Konservierungsmitteln, Bakteriziden, Fungiziden und ähnlichen auf Mikroorganismen wirkenden Substanzen verringert werden kann bzw. auf ihren Einsatz überhaupt verzichtet werden kann, wodurch sich zahlreiche Verwendungsmöglichkeiten ergeben.

## Beschreibung

Die Erfindung betrifft ein Verfahren und eine Vorrichtung zur Behandlung von Mikroorganismen bzw. eines Mikroorganismen enthaltenden Mediums sowie besondere Verwendungen des erfindungsgemäßen Verfahrens.

Um den Einsatz von Desinfektionsmitteln, Konservierungsstoffen, Bakteriziden, Fungiziden und anderen Mikroorganismen beeinflussenden Mitteln zu verringern und damit den Ernährungskreislauf, den Boden und das Abwasser zu entlasten, wird ständig nach neuen Mitteln und Verfahren gesucht, die eine Beeinflussung von Mikroorganismen ohne Nebenwirkungen zulassen.

Aufgabe der Erfindung ist es daher, eine Möglichkeit zu finden, Mikroorganismen nebenwirkungsfrei zu beeinflussen, so daß der Einsatz von Desinfektionsmitteln, Konservierungsmitteln, Bakteriziden, Fungiziden und ähnlichen auf Mikroorganismen wirkenden Substanzen verringert werden kann bzw. auf ihren Einsatz überhaupt verzichtet werden kann.

Die Aufgabe wird dadurch gelöst, daß bei dem eingangs genannten Verfahren ein in seiner elektromagnetischen Struktur durch Änderung der magnetischen Kernresonanzeigenschaften und durch Ausbildung von supramolekularen Komplexen zwischen den Molekülen in seinem Schwingungszustand geändertes Wasser oder Medium indirekt auf das die Mikroorganismen enthaltende Medium einwirkt oder diesem direkt zugesetzt wird. Dabei kann erfindungsgemäß eine Vorrichtung zum Einsatz kommen, die aus einem Doppelmantelgefäß besteht, wobei das in seiner elektromagnetischen Struktur und in seinem Schwingungszustand veränderte Wasser oder Medium im äußeren Mantel eingefüllt ist und damit ohne direkte Berührung auf die Mikroorganismen im Medium einwirkt, daß sich im inneren Mantel befindet oder den inneren Mantel durchströmt.

Die primäre Aktivierung von Flüssigkeiten, wobei durch Umwandlung eines Teiles der inneren Energie unter Abkühlung und Eintrag von Behandlungsenergie ein höherer Energieinhalt aufgeprägt wird, ist an sich bekannt. Es ist auch gelungen, Wasser in seiner elektromagnetischen Struktur so umzuwandeln, daß sowohl durch Modifikation der magnetischen Kernresonanzeigenschaften (Änderung der Spin-Spin-Kopplungskonstanten) als auch mittels Induktion der elektro-magnetische Schwingungszustand durch Ausbildung von supermolekularen Komplexen zwischen einzelnen Wassermolekülen modifiziert wurde. Diese elektromagnetischen Schwingungen sind verantwortlich für das Entstehen eines elektromagnetischen Feldes von definierter Frequenz, Phase und Amplitude, welche sich in alle Richtungen gleichmäßig ausbreitet.

Überraschenderweise hat es sich gezeigt, daß größere Partikel und Molekülgruppen in einem derartigen elektromagnetischen Feld in kleine Molekülgruppen zerlegt werden, wodurch unter anderem die Viskosität verringert wird, Schadstoffmoleküle aufgelöst werden können und dgl. mehr. Höchst überraschend ist auch, daß der energetische Zustand der aktivierten Flüssigkeiten beibehalten wird, das heißt, nicht abklingt.

Derart aktivierte Flüssigkeiten wurden bisher in einem Verfahren zur Verminderung des Kraftstoffverbrauches und der Abgase bei Brennkraftmaschinen, das in der EP-A 389 888 beschrieben ist, eingesetzt. Bei dieser bekannten Anordnung wird in der Kraftstoffzuführung zur Verbrennungskammer ein Aktivator angeordnet, der eine von Kraftstoff durchströmte Kammer und mindestens eine mit einem ruhenden Medium gefüllte Kammer aufweist, bei dem die elektromagnetische Struktur durch Änderung der magnetischen Kernresonanzeigenschaften und der Schwingungszustand durch Ausbildung von supermolekularen Komplexen zwischen den Molekülen geändert ist.

Durch das eingangs erwähnte elektromagnetische Feld erfolgt eine Beeinflussung auf andere Flüssigkeiten, wobei die Wirkung durch Wandungen hindurchgeht und dadurch Molekülgruppen verkleinern kann, so daß Schadstoffmoleküle aufgelöst werden. Dadurch findet auch eine vollkommenere Verbrennung von Kraft- und Heizstoffen mit einer drastischen Reduzierung der Schadstoffemission statt. Durch die vollkommenere Verbrennung kann zusätzlich Kraftstoff eingespart werden.

Völlig überraschend ist, daß durch die von der Flüssigkeit, die in in obengenannter Weise aktiviert wurde, ausgehenden Eigenschaften die im Medium enthaltenen Mikroorganismen verändert werden.

Die im Medium enthaltenen Mikroorganismen bestehen meist aus vielen Einzelindividuen. Diese schließen sich zu sogenannten Großkolonien zusammen, weil sie daraus ökologische Vorteile ziehen.
Überraschender Weise wurden durch die von der aktivierten Flüssigkeit ausgehenden Eigenschaften die Großkolonien in viele, winzige Einzelkolonien zerschlagen. Dadurch kommt es im Medium zu einem Anstieg der Gesamtkeimzahl.

Der Vorteil des erfindungsgemäßen Verfahrens liegt nun darin, daß diese durch Zerschlagung gebildeten Einzelkolonien gegenüber Desinfektionsmitteln und anderen mikrobizid wirkenden Medien, aber auch gegenüber Temperaturerhöhungen wesentlich empfindlicher sind als die Großkolonien. Die Einzelkolonien können demnach schon bei geringsten Konzentrationen von Desinfektionsmitteln in ihrem Wachstum gehemmt (Bakteriostase) bzw. völlig abgetötet (Bakterizide) werden. Dadurch ergibt sich eine deutliche Einsparung von Desinfektionsmitteln.

Eine Wachstumshemmung bzw. eine Abtötung von Mikroorganismen kann dadurch erfolgen, daß die einmal zerschlagenen Großkolonien immer wieder im Kreislauf durch die erfindungsgemäße Vorrichtung fließen.

Bekanntlich kann die Zerschlagung von Großkolonien in Einzelkolonien auch durch Ultraschall bewirkt werden. In Abhängigkeit von der Einwirkungszeit werden alle vorhandenen Großkolonien zerschlagen und damit ein Keimzahloptimum erreicht. Bei weiterer Einwirkung dieser hochfrequenten Schallenergie kommt es letztlich sogar zu einem Abtöten der Mikroorganismen. Der Kurvenverlauf in einem Diagramm (Ordinate: Keimzahl. Abszisse: Einwirkungszeit) entspricht einer Gauß'schen Glockenkurve.

Die vom ruhenden Medium, d.h. von der aktivierten Flüssigkeit der erfindungsgemäßen Anordnung ausgehenden Eigenschaften unterscheiden sich aber deutlich von der Wirkungsweise des Ultraschalls:
Wird die beschallte Probe unmittelbar nachher bakteriologisch untersucht, dann ist sofort eine Keimzahlerhöhung durch die Bildung von Einzelkolonien nachweisbar. Wird dagegen ein fließendes Medium durch die erfindungsgemäße Vorrichtung geleitet und anschließend sofort bakteriologisch untersucht, ist zunächst keine Veränderung gegenüber dem Ausgangsstadium feststellbar, das heißt, die Großkolonien sind in noch unzerschlagener Weise vorhanden.

Läßt man aber das fließende Medium außerhalb der Anlage 24 bis 96 Stunden stehen, dann sind nach dieser Verweilzeit bakteriologisch die Bildung von Einzelkolonien und die damit verbundene Keimzahlerhöhung nachweisbar. Daraus wird geschlossen, daß die vom ruhenden Medium ausgehende Eigenschaft nicht in Form von Energie auf die Mikroorganismen einwirkt, sondern in Form einer energielosen Informationsübertragung.
Vom ruhenden Medium wird demnach keine Energie abgestrahlt. Nur damit ist zu erklären, warum nach 11 Jahren Beobachtungszeit (die Experimente wurden 1983 begonnen) die damals gebaute Vorrichtung heute immer noch funktioniert.

Im Europäischen Patent 389 888 wird angeführt, "daß größere Partikel und Molekülgruppen in einem derartigen elektromagnetischen Feld in kleinere Molekülgruppen zerlegt werden".

Damit wird erklärt, warum es bei Kraft- und Heizstoffen zu einer drastischen Reduzierung der Schadstoffemission kommt. Keinesfalls ist damit die hier erfindungsgemäß angeführte Zerschlagung von mikrobiologischen Großkolonien in empfindlichere Einzelkolonien gemeint, da Kraft- und Heizstoffe frei von Mikroorganismen sind.

In dem obengenannten EP-Patent meint man mit der Zerschlagung größerer Partikel und Molekülgruppen in kleinere Molekülgruppen die physikalische Umwandlung von in Heizöl enthaltenen Verbindungen, mit dem Ziel, eine bessere Verbrennung und eine Senkung von unerwünschten Emissionen zu erreichen.

Bei der gegenständlichen Erfindung geht es ausschließlich um die Zerschlagung von Mikroorganismen - also der Umwandlung von Großaggregaten in Einzelzellen.

Durch die erfindungsgemäß nachgewiesene Einwirkung des ruhenden Mediums auf Mikroorganismen ist es erstmals möglich geworden, die von diesem Medium ausgehende Wirkung auch bildhaft mit Hilfe bakteriologischer Methoden nachzuweisen.

Diese Veränderung bei Mikroorganismen wird auch dann erreicht, wenn das in seiner elektromagnetischen Struktur veränderte Wasser direkt einem Wasser zugesetzt wird, das Mikroorganismen enthält. Diese Anordnung, die dabei eingesetzte bakteriologische Nachweistechnik und die erhaltenen Ergebnisse sollen in Form eines Beispieles dargelegt werden:

Eingesetzt wird ein frei aus der Wasserleitung fließendes Trinkwasser, das bei guter Qualität eine Keimzahl von etwa 10 koloniebildenden Einheiten pro Milliliter enthält. Als Keimzahl oder Koloniezahl wird entsprechend der Trinkwasserverordnungen der einzelnen Länder allgemein die Zahl der mit 6- bis 8-facher Lupenvergrößerung sichtbaren Kolonien definiert, die sich aus den in 1 ml des zu untersuchenden Wassers befindlichen Bakterien in Plattengußkulturen oder nach dem Membranfilterverfahren mit nährstoffreichen, peptonhaltigen Nährböden (1% Fleischextrakt, 1% Pepton) bei einer Bebrütungstemperatur von 20 +2°C sowie 36 +1°C nach 44 +4 Stunden Bebrütungsdauer bilden.
Beim Membranfilterverfahren werden zur Bestimmung der Keimzahl bakterienundurchlässige Membranfilter mit einer Porengröße zwischen 0,2 und 0,45 µm und einem Durchmesser von 55 mm eingesetzt. Das zu analysierende Wasser wird durch diese Membranfilter hindurchfiltriert. Die in diesem Wasser enthaltenen Bakterien und Pilze bleiben auf dem Filter zurück. Im Anschluß daran wird dieses Filter auf einen Nährboden aufgelegt, wie oben beschrieben bebrütet und danach die sich gebildeten Bakterienkolonien ausgezählt.
Das Membranfilterverfahren ist ein genormtes Verfahren nach DIN 38.411-K5 "Bestimmung vermehrungsfähiger Keime mittels Membranfilterverfahren" und entspricht dem Deutschen Einheitsverfahren zur Wasser-, Abwasser- und Schlammuntersuchung (DEV).

Vom Trinkwasser wird zunächst ein bakteriologischer Ausgangswert mit Hilfe der oben beschriebenen Technik bestimmt. Danach wird zu einem Liter dieses Trinkwassers 1 ml eines sterilfiltrierten Wassers zugegeben, das in seiner elektromagnetischen Struktur im Sinne des EP 389 888 verändert wurde.

Für die Blindwertbestimmung wird in einem zweiten Gefäß 1 Liter dieses Trinkwassers ohne jeden Zusatz eingefüllt. Beide Gefäße werden nun 12 bis 96 Stunden zugedeckt stehengelassen. Dabei ist wichtig, daß beide Gefäße mindestens 10 m voneinander entfernt stehen, damit vom beimpften Gefäß kein Einfluß auf das Blindwertgefäß erfolgen kann.
Nach dieser Zeit wird erneut eine Keimzahlbestimmung von beiden Gefäßen mit Hilfe des Membranfilterverfahrens durchgeführt. Dabei zeigt sich, daß die Keimzahl der bei 21°C 44 Stunden lang bebrüteten Agarplatten der beimpften Lösung im Vergleich zum Blindwert um etwa 90% gesunken ist. Das gleiche gilt für die bei 37°C über 24 Stunden bebrüteten Platten.

Wird nun die Agarplatte länger als 44 Stunden bei 21°C bebrütet, dann treten überraschenderweise neue und zunächst winzig kleine Kolonien auf, die nach längstens 120 Stunden Bebrütungszeit voll entwickelt sind. Im Vergleich zu den Kolonien des unbehandelten Wassers sind diese neu auftretenden Kolonien in ihrem Aussehen verändert: Sie sind entweder gelb oder opak weiß eingefärbt, sehr viel kleiner und eine Kolonie gleicht der anderen, das heißt, die Artenvielfalt im Aussehen der Kolonien, wie sie das unbehandelte Wasser zeigt, ist verlorengegangen. Die Keimzahl nach 120 Stunden Bebrütungszeit kann über tausend KBE/ml betragen.

Wird die Einwirkungszeit (in diesem Beispiel 12 Stunden) auf mehrere Tage ausgedehnt, dann sind meist nach drei Tagen überhaupt keine Ausgangskolonien mehr nachweisbar, das heißt, innerhalb der ersten 44 Stunden Bebrütungszeit bei 20 bis 22°C zeigen sich meist keine Kolonien mehr. Diese entstehen erst bei längerer Bebrütungszeit in der oben beschriebenen Form als Tochterkolonien. Bei 37°C Bebrütungstemperatur tritt auch bei längerer Bebrütungszeit keine Koloniebildung mehr auf, da die gebildeten Einzelkolonien sehr temperaturempfindlich sind.

Erfindungsgemäß wird diese mikrobiologische Veränderung wie folgt erklärt:
Mikroorganismen und speziell Bakterien leben in einem Medium, wie z.B. Wasser, selten als Einzelindividuum. Der Zusammenschluß mehrerer Einzelindividuen zu einem Muttergebilde hat Vorteile hinsichtlich Energienutzung, Substratverwertung und Überlebenschance. Werden solche Muttergebilde mit einem in seiner elektromagnetischen Struktur veränderten Wasser in Kontakt gebracht, dann kann man nach einer oben angegebenen Verweilzeit nachweisen, daß aus einer Mutterkolonie eine Vielzahl von Tochterkolonien gebildet wird.

Erfindungsgemäß konnte auch nachgewiesen werden, daß diese Tochterkolonien eine wesentlich schlechtere Überlebenschance haben und durch Desinfektionsmittel in geringsten Konzentrationen abgetötet werden. Diese Konzentrationen sind wesentlich geringer als jene, die zum Abtöten der Mutterkolonien notwendig sind.
Die geringeren Überlebenschancen der Tochterkolonien zeigen sich auch darin, daß diese eine deutlich längere Bebrütungszeit benötigen (nämlich mehr als 40 Stunden), um Kolonien auf Agarplatten zu bilden. Diese Tochterkolonien sind auch wesentlich temperaturempfindlicher als die Mutterkolonien. Bei 37°C sterben sie ab.
Die Bildung derart empfindlicher Tochterkolonien wird auch dann beobachtet, wenn bakterienhältige Flüssigkeiten mit UV-Strahlen belastet werden, die Dosis aber so gewählt wird, daß eine Abtötung noch nicht erfolgt. Dies ist aus solchen Untersuchungen bekannt, bei denen die UV-Lampen durch Belagsbildung in ihrer Wirkung geschwächt waren. Durch subletalen Zusatz von H₂O₂ kann ebenfalls eine Bildung von Tochterkolonien beobachtet werden. Auch diese Beobachtungen stammen von Berichten, bei denen die Konzentration des H₂O₂ durch eine nicht ausreichende Stabilität zu gering war, um abtötend zu wirken.
Daß Tochterkolonien auch durch ein in seiner elektromagnetischen Struktur verändertes Wasser gebildet werden können, ist dagegen überraschend und neu.

Für den technischen Einsatz wird eine Vorrichtung gewählt, bei der das in seiner elektromagnetischen Struktur veränderte Wasser nicht direkt zugesetzt wird. Vielmehr wird dieses in einen starren Doppelmantel eingefüllt und verschlossen. Dieser Doppelmantel umschließt ein Rohr, durch welches nun das zu behandelnde Medium mit einer bestimmten Geschwindigkeit fließt.
Auch bei dieser Vorrichtung wurden, wie oben beschrieben, die gleichen Phänomene beobachtet: Abnahme der Mutterkolonien, Bildung von Tochterkolonien.
Wird das zu behandelnde Medium in dieser Anordnung im Kreislauf geführt, dann ist es möglich, nach einer entsprechenden Verweilzeit die Tochterkolonien auch ohne den Zusatz von Desinfektionsmitteln zu reduzieren oder letztlich sogar vollständig abzutöten.

Aus dieser Wirkung des in seiner elektromagnetischen Struktur veränderten Wassers auf Mikroorganismen läßt sich eine Fülle von praktischen Anwendungen ableiten, die nachfolgend in Form von Beispielen beschrieben werden sollen:

### Beispiel 1: Anwendung im Bereich Schwimmbad.

Ein privates Schwimmbad mit den Abmessungen 12 m Länge, 4 m Breite, 1,50 m Tiefe, Volumen demnach 72 m³, wurde am 30. Juli 1994 beprobt, um den bakteriologischen Ist-Stand zu erheben.
Ergebnisse:
95 KBE pro ml + 1 Pilzkolonie auf Plate Count-Agar, Bebrütung 22°C, 48 Stunden.
5 KBE pro ml bei einer Bebrütung von 24 Stunden bei 37°C. pH-Wert 8,2.
Chlorgehalt nicht nachweisbar. Aussehen des Wassers: noch klar, beginnender Algenwuchs an den Schwimmbadwänden. Bei eingeschalteter Umwälzpumpe wurde das Schwimmbadwasser mit insgesamt 20 ml eines in seiner elektromagnetischen Struktur veränderten Wassers direkt versetzt.

Bakteriologisches Ergebnis am 3.8.1994 (drei Tage nach der Beimpfung):
Keine Mutterkolonien mehr nach 48 Stunden Bebrütungszeit bei 22°C, dafür sind eine Vielzahl kleinster, mit freiem Auge gerade noch erkennbarer Kolonien entstanden. Nach 60 Stunden Bebrütungszeit lassen sich zirka 500 Tochterkolonien auszählen. Nach 90 Stunden Bebrütungszeit ist die Platte vollständig überwachsen und keine Einzelkolonien mehr zählbar. pH-Wert des Wassers: 8,0.

10.8.1994: Stark frequentierter Badebetrieb. pH-Wert 8,6. Das Badewasser trübt sich ein. Deutliche Algenbildung am Beckenboden. Bakteriologische Prüfung: 6 Mutterkolonien und eine nicht zählbare Zahl von Tochterkolonien. Um den Algenbewuchs zu stoppen, wird ein Desinfektionsmittel (Sauerstoffabspalter auf Essigsäurebasis) zugegeben und der pH-Wert auf 7,0 eingestellt. Desinfektionsmittelkonzentration im Schwimmbadwasser: 0,0005%!

12.8.1994: Das Wasser ist wieder vollständig klar. Bakteriologische Prüfung: Keimzahl Null.

22.8.1994: Keimzahl Null. Nach wie vor starker Badebetrieb.
pH-Wert 7,1. Wasser klar.

20.9.1994: pH-Wert 7,8. Das Wasser ist trüb, deutlicher Algenbewuchs.
Keimzahl: 3 Mutterkolonien und unzählbare Tochterkolonien.

Zusammenfassendes Ergebnis: Durch Zusatz einer äußerst geringen Menge (20 ml für 73 m³) eines in seiner elektromagnetischen Struktur veränderten Wassers zum Schwimmbadwasser wurde bewirkt, daß die Keimzahlen während einer Beobachtungszeit von zwei Monaten um 95% reduziert wurden. Die dabei entstandenen Tochterkolonien waren gegen das zugesetzte chlorfreie Desinfektionsmittel auf Essigsäurebasis so empfindlich, daß eine 0,0005%ige Endkonzentration im Schwimmbad genügte, die Keimzahl auf Null zu reduzieren (weder Mutter- noch Tochterkolonien nachweisbar) und diesen Status trotz eines intensiven Badebetriebes (sehr heißer Sommer 1994) und trotz 26°C Wassertemperatur 14 Tage aufrecht zu erhalten.
Selbst nach 2-monatiger Beobachtungszeit konnte die an das Wasser abgegebene Information durch das in seiner elektromagnetischen Struktur veränderte Wassers an Hand der Vielzahl der vorhandenen Tochterkolonien immer noch nachgewiesen werden. Das Algenwachstum konnte durch den Zusatz dieses Wassers nicht verändert werden.

Dieser Versuch über 50 Tage zeigt die Auswirkungen und Vorteile des Verfahrens:
- Verzicht auf chlorhaltige Desinfektionsmittel
- Reduktion des Desinfektionsmittelzusatzes um mehr als 70%
- Vorrangiger Einsatz von algizid wirkenden Mitteln.

Trotz eines intensiven Badebetriebes (6 Personen, täglich etwa 4 Badegänge) und Wassertemperaturen bis 26°C stieg nach Beimpfung des Wassers die Keimzahl nicht. Nur der Algenbewuchs wurde durch die Beimpfung nicht gehemmt. Allein dieser machte einen gezielten und in seiner Menge deutlich reduzierten Einsatz chemischer Mittel notwendig.

Wird das Schwimmbadwasser nicht direkt beimpft, sondern ein Doppelmantelgefäß - wie oben beschrieben - in den Kreislauf der Umwälzpumpe eingebaut, dann ist die Bildung von Tochterkolonien nach 7 bis 14 Tagen nachweisbar. Die schnellste und zugleich nachhaltigste Wirkung wird erreicht, wenn das Schwimmbadwasser einerseits direkt beimpft und andererseits ein Doppelmantelgefäß in den Kreislauf der Umwälzpumpe eingebaut wird.

### Beispiel 2: Geruchsbeseitigung bei Gülle sowie Anhebung des Düngewertes.

Die von Säugetieren abgesetzte, frische Gülle hat einen pH-Wert zwischen 6,5 und 7,5 und ist geruchlos. Durch den hohen Harnstoffgehalt ist die Gülle ein sehr guter, bakteriologischer Nährboden. Bei der bakteriellen Hydrolyse wird zunächst Harnstoff in Ammoniak und Kohlendioxid umgewandelt. 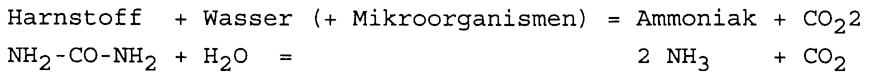
Erst durch diese mikrobielle Umwandlung beginnt die Gülle nach Ammoniak zu stinken. Gleichzeitig steigt der pH-Wert auf 10 bis 12 an. Die Gülle ist in diesem Zustand stark alkalisch, damit pflanzenunverträglich und darf so nicht landwirtschaftlich ausgebracht werden.
Wird die ammoniakhaltige Gülle stark gerührt, um ihr Sauerstoff zuzuführen, dann wandelt sich das Ammoniak mit Hilfe von Mikroorganismen (Nitrifikanten) in das pH-neutrale Nitrat um. Durch diese Nitrifizierung sinkt der pH-Wert wieder in den Neutralbereich, die Gülle stinkt nicht mehr und ist jetzt optimal pflanzenverträglich mit hohem Düngewert. Versuche haben nun überraschenderweise gezeigt, daß durch direktes Beimpfen der Gülle mit einem in seiner elektromagnetischen Struktur erfindungsgemäß veränderten Wasser - oder durch Einhängen eines erfindungsgemäßen Doppelmantelstabes in die Güllegrube - oder durch Durchleiten der Gülle durch ein Doppelmantelgefäß (eingebaut in den Kreislauf der Gülle-Umwälzpumpe) - oder durch Kombination der angegebenen Methoden - die Nitrifizierung deutlich rascher abläuft als ohne Beeinflussung.

Bei Laborversuchen mit gleicher Ausgangsgülle konnte bei jenem Gefäß, in das ein Doppelmantelstab eingehängt war und das zusätzlich direkt beimpft wurde, im Vergleich zum Blindwert folgendes festgestellt werden:
- Der pH-Wert fiel signifikant schneller in den Neutralbereich.
- Die CO₂-Entwicklung war deutlich stärker und
- der Nitratgehalt stieg signifikant rascher an.

Vorteile daraus:
- Die Zeitspanne, in der Geruchsbildung möglich ist, wird drastisch gesenkt.
- Bei praktischen Versuchen war dies ein für den Anwender selbst feststellbares Erfolgserlebnis: Die Gülle stinkt nicht mehr!
- Die Zeitspanne bis zur Ausbringung der Gülle wird verkürzt.

Besonders erfolgreich waren jene praktischen Versuche, bei denen die Gülle
1. umgepumpt,
2. eine direkte Beimpfung vorgenommen und
3. ein Doppelmantelstab permanent eingebaut war.

### Beispiel 3:

Verhinderung von Geruchsbildung, bakterieller Belagsbildung und überschießender Bakterienvermehrung in Leitungen mit nährstoffreichen Medien am Beispiel zahnärztlicher Stühle.

Zahnärztliche Stühle haben eine Fülle von Wasserzufluß-, Abfluß- und Absaugleitungen. Speichel und Blut der Patienten machen die Abwässer besonders nährstoffreich. Dadurch können sich Mikroorganismen im Leitungssystem zahnärztlicher Stühle besonders stark vermehren. Dies kann zu Geruchsbildung führen. Gleichzeitig steigt das Infektionsrisiko.

Das Wasser zum Befüllen des Mundspülglases wird aus der Trinkwasserleitung automatisch entnommen, in einem Vorratsbehälter auf 37°C angewärmt und bei Bedarf in das Mundspülglas gepumpt. Aus der Literatur ist bekannt, daß gerade dieses Mundspülwasser durch das Warmhalten während der Behandlungszeiten und das stagnierende Stehen über Nacht hochgradig mit Pseudomonas aeruginosae verkeimt ist. Infektionen und Veränderungen der Mundflora bei Patienten sind die Folge.

Um das Infektionsrisiko zu senken, versucht man, diesem Wasser ein Desinfektionsmittel zuzusetzen, z.B. eine stabilisierte 0,7%ige Wasserstoffperoxidlösung. Dies hat den Nachteil, daß das Spülwasser einen unangenehmen Geschmack (nach Gurgelwasser) bekommt. Bei bakteriologischen Untersuchungen zeigte sich, daß durch Unterdosierung - sei es durch mangelhafte Stabilisierung des H₂O₂-Konzentrates oder durch einen fehlerhaften Dosiermechanismus - der Bakteriengehalt nur unwesentlich gesenkt wurde. Pseudomonaden hatten durch die Unterdosierung oftmals Resistenzen entwikkelt und waren gegen H₂O₂ unempfindlicher geworden. Eine dauerhafte Problemlösung konnte bisher nicht gefunden werden. Ein Höherdosieren des Desinfektionsmittels konnte zwar die Keimzahl im Mundspülwasser senken, war aber vom Geschmack her für den Patienten unzumutbar.

Dazu kommt, daß vor allem Pseudomonaden in strömenden Systemen bakterielle Beläge bilden. Diese aus mehreren Zelluloseeinheiten bestehenden, klebrigen Beläge haften sehr gut an der Wand und bilden gleichzeitig eine Brutstätte für neue Pseudomonaden. Dieser Belag hat für die Mikroorganismen auch den Vorteil, daß sie von der Wirkung der zugesetzten Desinfektionsmittel geschützt werden.

Ebenfalls aus der Literatur ist bekannt, daß Rohrleitungen, in denen derartige bakterielle Beläge vorhanden sind, nicht mehr desinfiziert werden können. Nur durch eine mechanische Entfernung der Beläge können auch die Bakterien dauerhaft beseitigt werden.

Bei einem Zahnarzt wurde die erfindungsgemäße Anordnung in Form eines Doppelmantel-Durchflußgerätes in jene Trinkwasserleitung eingebaut, die zum heizbaren Vorratsbehälter für das Mundspülwasser führt. Zusätzlich wurde dieser Vorratsbehälter mit einigen Tropfen des in seiner elektromagnetischen Struktur veränderten Wassers beimpft. Bereits drei Tage nach dieser Beimpfung konnte bakteriologisch die Wirkung festgestellt werden: Es waren keine Mutterkolonien mehr nachweisbar. Die Tochterkolonien waren nach einer Bebrütungszeit von 60 Stunden bei 21°C nachweisbar. Ihre Keimzahl betrug etwa 1500/ml.

7 Tage nach Versuchsbeginn wurde der heizbare Vorratsbehälter geöffnet und festgestellt, daß sich am Boden dieses Gefäßes abgelöste, bakterielle Beläge und Kalkstein angesammelt hatten. Diese wurden entfernt und der Vorratsbehälter wieder geschlossen. In den nachfolgenden Wochen wurde eine stetige Abnahme der Keimzahl der Tochterkolonien beobachtet.

Zwei Monate nach Versuchsbeginn war die Keimzahl der Tochterkolonien unter 100 KBE/ml gesunken. Das Wasser hatte seinen natürlichen Trinkwassergeschmack. Auf die Zugabe von Desinfektionsmitteln konnte in der Folge völlig verzichtet werden.

Nach drei Monaten wurde der Vorratsbehälter wiederum geöffnet: Die Wandungen waren sauber und frei von Belägen. Am Boden wurde nur noch ein Rest von abgeblätterten Belägen und Kalk festgestellt und dieser entfernt. Mutterkolonien, so wie diese im zufließenden Trinkwasser festgestellt wurden, waren im Mundspülwasser des Patienten nicht mehr nachweisbar.

Neben dieser Sanierung des Trinkwasserbereiches einer zahnärztlichen Behandlungsanlage wurde auch versucht, die sonstigen wasserzuführenden bzw. abführenden Leitungen bakteriologisch zu sanieren. Dies betrifft auch die Absaugeinrichtungen.

Neuerdings werden durch gesetzliche Forderungen in den Abflußbereich von zahnärztlichen Behandlungsanlagen sogenannte Amalgamabscheider eingebaut. Dies sind Geräte, die verhindern sollen, daß Amalgamreste, wie sie beim Herstellen von Plomben oder beim Ausbohren alter Amalgamplomben anfallen, in das Kanalnetz eingespült werden. Insbesonders frisches Amalgam bzw. solches, das beim Ausbohren alter Amalgamplomben sehr fein verteilt wird, kann zu einem Anstieg des löslichen Quecksilbergehaltes im Abwasser führen. Amalgamabscheider können zum Beispiel durch eingebaute Zentrifugen das enthaltene Amalgam vom sonstigen Abwasser trennen. Das Amalgam wird in Vorratsbehältern gesammelt und diese werden nach Erreichen eines bestimmten Füllzustandes einem Recyclingverfahren zugeführt.

Aus hygienischer Sicht bedeutet der Einbau eines Amalgamabscheiders einen zusätzlichen Durchflußwiderstand. Die notwendigen Vorratsbehälter für Amalgam bewirken, daß Abwasser über Nacht in diesen Behältern stehen bleibt und daß sich darin enthaltene Mikroorganismen stark vermehren können. Diese starke Vermehrung führt gleichzeitig zu einer verstärkten Schleim- und Belagsbildung. Damit werden Schaltsensoren für den Amalgamabscheider überzogen und machen diesen funktionsunfähig. Auf der anderen Seite kann eine unangenehme Geruchsbildung in der Zahnarztpraxis entstehen. Beides ist unerwünscht.

Bisher hat man die Vermehrung von Mikroorganismen und die Bildung von Schleim und Belägen durch Zugabe von Desinfektionsmitteln bekämpft. Um Beläge verstärkt ablösen zu können, hat man oftmals stark alkalische Desinfektionsmittel eingesetzt (pH 11 bis 12). Die Belagsbildung wurde damit zwar reduziert. Diese stark alkalischen Lösungen sind aber auch in das Vorratsgefäß des Amalgamabscheiders gelangt, also dort wo die auszentrifugierten Amalgamreste aufbewahrt werden.

Durch die feine Oberfläche dieser Amalgamreste und durch die stark alkalischen Lösungen kam es zu einer erhöhten Löslichkeit für Quecksilber und damit wurde genau das Gegenteil erreicht, was man mit dem Einbau eines Amalgamabscheiders eigentlich verhindern wollte: Der Amalgamabscheider hat zwar das feste Amalgam abgeschieden, stark alkalische Desinfektionsmittel haben dagegen einen Teil des festen Amalgams wieder aufgelöst und damit gelöstes Amalgam in den Kanal eingetragen. Die Abwasseremissionsverordnungen begrenzen den Gehalt an löslichem Quecksilber im Abwasser mit maximal 0,01 mg/l. Diese Werte wurden bei weitem überschritten.

Zur erfindungsgemäßen Lösung dieses Problems wurden in alle wasserzuführenden Leitungen der zahnärztlichen Behandlungseinheit Doppelmantel-Durchflußgefäße eingebaut und zusätzlich der Amalgamvorratsbehälter der Abscheidevorrichtung direkt mit einem in seiner elektromagnetischen Struktur veränderten Wasser beimpft = Versuchsstuhl. Der zweite Stuhl des Versuchszahnarztes wurde wie bisher mit Desinfektionsmittel behandelt = Vergleichsstuhl. Die bakteriologische Untersuchung erfolgte einmal wöchentlich aus dem Wasser des Amalgamvorratsbehälters im Abscheidegerät.

Eine Woche nach Versuchsbeginn wurde beim Versuchsgerät festgestellt, daß sich im Vorratsgefäß des Amalgamabscheiders, oberhalb des schweren Amalgamrückstandes, eine Fülle von organischen Belägen abgesetzt hat. Diese haben sich offenbar in letzter Zeit abgelöst. Auch der Amalgambehälter selbst zeigte an seinen Wandungen entsprechende Ablösespuren dieser Beläge. Die abgelösten Beläge wurden entfernt. Die erste bakteriologische Prüfung ergab eine deutliche Zunahme von Tochterkolonien, daneben waren aber noch vereinzelt Mutterkolonien nachweisbar.

Der Vergleichsstuhl hingegen zeigte keinen vermehrten Anfall von abgelösten Belägen. Unter den Mutterkolonien waren etwa 100 KBE/ml fluoreszierender Pseudomonaden (Fluoreszenz bei 366 nm).

Vier Wochen nach Versuchsbeginn waren die Leitungen des Versuchsstuhles im Inneren vollständig sauber, insbesondere die Wandungen des Amalgamvorratsbehälters und die Sensoren im Zuflußbereich. In dieser Zeit kam es zu keinem Ausfall der Abscheideanlage und auch zu keiner Geruchsbildung. Nach 4 Wochen waren keine Mutterkolonien mehr nachweisbar. Die Tochterkolonien hatten eine Keimzahl von 1000/ml.

Das Vergleichsgerät zeigte dagegen einen leichten Anstieg der Mutterkolonien und die übliche Belagsbildung. Dosiert wurden täglich 20 g eines pulverförmigen Desinfektionsmittels auf der Wirkstoffbasis eines Sauerstoffabspalters.

Diese Desinfektionslösung wurde 1%ig zugesetzt, pH-Wert 10,0. Quecksilbergehalt im Abwasser: 0,03 bis 0,1 mg/l.

Nach 6-monatiger Versuchszeit blieb das Ergebnis im Versuchstuhl konstant: keine neue Belagsbildung, keine störende Geruchsbildung und eine starke Bildung von Tochterkolonien mit weit über 1000 KBE/ml. Eine zusätzliche Dosierung von Desinfektions-mitteln war nicht notwendig.

Durch die erfindungsgemäße Vorrichtung und das hier beschriebene Verfahren lassen sich bei zahnärztlichen Behandlungseinheiten folgende Vorteile aufzeigen:
1. Weitgehender Verzicht auf den Zusatz von Desinfektionsmitteln.
2. Keine Auflösung von abgeschiedenem Quecksilber, wie dies z.B. durch alkalische Desinfektionsmittel geschieht.
3. Keine Geruchsbildung aus den Schläuchen heraus.
4. Keine Belagsbildung und damit Gewährleistung eines störungsfreien Betriebes der Behandlungseinheit, insbesondere des Amalgamabscheiders.

### Beispiel Nr. 4:

Verhinderung der bakteriologischen Verkeimung und der Geschmacksänderung von Trinkwasser, das bei Tropenreisen in Plastik-Vorratsbehältern mitgeführt wird.

Die Reinhaltung von Trinkwasser bei Tropenreisen ist ein generelles Problem. Um nicht auf manchmal zweifelhafte Trinkwasserquellen angewiesen zu sein, besteht besonders bei Autoreisen der Wunsch, heimisches Trinkwasser in guter Qualität in Plastikbehälter abzufüllen, um dieses für die gesamte Reise vorrätig zu haben. Da aber auch bestes Trinkwasser nicht frei von Mikroorganismen ist, kann es besonders durch erhöhte Temperatur und Sonneneinstrahlung, aber auch begünstigt durch die Kunststoffbehälter und eventuell darin enthaltene Weichmacher zu einer starken Verkeimung kommen. Sogar Algenbildung ist möglich. Dies alles kann zu einer Genußuntauglichkeit des Trinkwassers führen.

Es sind verschiedene Verfahren bekannt, Trinkwasser für diese Zwecke haltbar zu machen.
Eines dieser Verfahren ist die Zugabe eines unter dem Warenzeichen "Micropur" der Firma Katadyn Deutschland-GmbH angebotenen Produktes. Es besteht aus einem lichtbeständigen, wasserlöslichen Natrium-Silber-Chlorid-Komplex. Dieses Pulver löst sich in Wasser vollständig auf. Nach Dissoziation des Natriumchlorides bewirkt das freigesetzte Silber die Entkeimung des Trinkwassers. Die dafür notwendige Konzentration beträgt 1 g Pulver auf 100 l Wasser. Ist eine vollständige Desinfektion beabsichtigt, sind 10 g Pulver auf 100 l Wasser zu dosieren. Die Einwirkungszeit wird mit 1 bis 2 Stunden angegeben.

Eine zweite Möglichkeit ist die Filtration des Wassers durch keimdichte Membranfilter. Dazu wird von der gleichen Firma eine Handpumpe mit entsprechenden Filtern angeboten.

Bei allen Vorteilen, die diese Produkte bieten, ist doch nicht zu übersehen, daß dem Wasser ein Fremdstoff in Form eines Silbersalzes zugesetzt wird. Beim Filtrationsverfahren ist stets ein Gerät mitzuführen und die Filtration selbst relativ mühsam. Dazu kommt, daß die einmal verwendeten Filter verkeimen können - deshalb wird auf einen oftmaligen Filterwechsel Wert gelegt.

Beim gegenständlichen Versuch ging es darum festzustellen, ob durch den Zusatz von sterilfiltriertem Wasser, das in seiner elektromagnetischen Struktur und in seinem Schwingungszustand verändert wurde, zum Wasser der Vorratstanks die Genußtauglichkeit des Trinkwassers auch unter tropischen Bedingungen erhalten werden kann.

Für einen vierwöchigen Urlaub in der nördlichen Sahara wurden für zwei Reisende 12 Kunststoffbehälter mit je 10 Liter einwandfreiem, quellfrischem Wasser befüllt.

Die bakteriologische Untersuchung dieses Wassers ergab folgende Werte:
Keimzahl bei 22°C Bebrütungszeit unter 5 KBE/ml.
Keimzahl bei 37°C Bebrütungszeit unter 2 KBE/ml.
Abwesenheit von Escherichia coli, coliforme Bakterien und Enterokokken.

Die Kunststoffkanister bestanden aus opaktransparentem Polypropylen mit selbstdichtendem Schraubverschluß.
Zu jedem dieser Gefäße wurde 1 ml eines sterilfiltrierten (0,2 µm) und in seiner elektromagnetischen Struktur verändertes Wasser zugesetzt, die Behälter verschlossen und umgeschüttelt. Durch die Anfahrtszeit war sichergestellt, daß eine Einwirkungszeit von mindestens 3 Tagen gewährleistet war.
Die Wasserbehälter wurden aus Platzgründen auf dem Dachträger des Autos transportiert und waren demnach voll der Sonne und den Außentemperaturen ausgesetzt. Es wurden Wassertemperaturen bis 42°C gemessen.

Nach 4-wöchigem Aufenthalt unter den genannten Bedingungen wurde von den Reisenden folgendes berichtet: Der Geschmack des Wassers war - abgesehen von der Wärme - unverändert. Der Geschmack konnte vor allem nach einer kühlen Nacht am Morgen besonders sicher beurteilt werden. In keinem der 12 Plastikbehälter konnte Algenbewuchs festgestellt werden. Fremdwasser wurde in die Behälter nicht nachgefüllt!

Die nach vier Wochen in jedem Behälter vorhandene Restwassermenge (zirka 50 ml) wurde ebenfalls bakteriologisch untersucht und folgendes festgestellt:
Nach 48 Stunden Bebrütungszeit bei 22°C null Keime.
Bei 120 Stunden Bebrütungszeit bei 22°C zirka 30 kleinste Tochterkolonien.

Dies beweist eine gute Wirksamkeit des zugesetzten und in seiner elektromagnetischen Struktur veränderten Wassers. Bei 37°C Bebrütungstemperatur wurden keine koloniebildenden Einheiten festgestellt.

Wesentlich für die Beurteilung dieses Praxisversuches sind zwei Kriterien:
- Bei 22°C Bebrütungstemperatur sind innerhalb einer Bebrütungszeit von 48 Stunden keine Kolonien auf der Agar-Platte entstanden. Winzige Tochterkolonien wurden erst nach 120 Stunden Bebrütungszeit gesehen. Auch hier war die Koloniezahl mit zirka 30 stark vermindert.
- Bei 37°C Bebrütungstemperatur sind weder nach 48 Stunden noch nach 120 Stunden Bebrütungszeit Kolonien auf der Agar-Platte entstanden. Krankheitserreger und alle hygienisch bedenklichen Keime sind auf die Körpertemperatur des Menschen adaptiert. Für die Beurteilung der hygienischen Qualität des Wassers ist gerade diese Bebrütungstemperatur sehr wesentlich.

Grenzwerte für Trinkwasser laut Trinkwasserverordnung:
maximal 100 KBE/ml bei 22°C nach 48 Stunden und
maximal 100 KBE/ml bei 37°C nach 48 Stunden.

Zur genauen Darstellung des Keimzahlverlaufes nach Versetzen eines Trinkwasser mit einem sterilfiltrierten Wasser, das in seiner elektromagnetischen Struktur verändert wurde, ist im Labor der folgende Versuch durchgeführt worden:
Das gleiche Trinkwasser, das für die oben beschriebene Tropenreise zum Einsatz kam, wurde bakteriologisch voruntersucht und danach 1 ml eines in seiner elektromagnetischen Struktur veränderten Wassers zu 10 Liter Trinkwasser zugesetzt. Eine aliquote Probe davon wurde in eine 1 Liter-Flasche gefüllt, die ebenfalls aus Polypropylen gefertigt war. Nach 3 Tagen Stehzeit bei Raumtemperatur wurde wiederum eine bakteriologische Untersuchung durchgeführt und dabei etwa 2300 Tochterkolonien bei einer Bebrütungstemperatur von 22°C nach 120 Stunden festgestellt. Nach 48 Stunden bei 22°C und nach 48 Stunden bei 37°C haben sich keine koloniebildenden Einheiten gezeigt.

Nach diesen drei Tagen wurde die 1 Liter-Flasche bei 37°C in den Brutschrank gestellt und nach jeweils einer Woche wieder bakteriologische Untersuchungen durchgeführt. Folgende Keimzahlen für Tochterkolonien wurden gefunden:
Nach einer Woche 250, nach zwei Wochen 100, nach drei Wochen 50 und nach vier Wochen 10 KBE/ml. Bebrütung 22°C, 120 Stunden.

Dieser Versuch zeigt, daß die gebildeten Tochterkolonien temperaturempfindlich sind. Bereits nach einer Woche Stehzeit bei 37°C konnten um 90% weniger koloniebildende Einheiten gezählt werden als zu Beginn der Temperaturbelastung.

Den Keimzahlverlauf der parallel mitgezogenen Blindprobe (reines Trinkwasser ohne Zusatz) zeigt die nachfolgende Tabelle:

| Keimzahlverlauf einer Trinkwasserprobe bei 37°C mit (Probe) und ohne (Blindwert) Zusatz | | | | |
|---|---|---|---|---|
| Zeit | | Anzahl der koloniebildenden Einheiten (KBE) | | |
| | | nach 48 h bei 22°C | nach 120 h bei 22°C | nach 48 h bei 37°C |
| nach 3 Tagen bei Raumtemperatur | Probe | 0 | 2300 | 0 (!) |
| | Blindwert | 5 | 5 | 2 |
| nach 1 Woche bei 37°C | Probe | 0 | 250 | 0 (!) |
| | Blindwert | 3 | 1 | 5 |
| nach 2 Wochen bei 37°C | Probe | 0 | 100 | 0 (!) |
| | Blindwert | 2 | 4 | 8 |
| nach 3 Wochen bei 37°C | Probe | 0 | 50 | 0 (!) |
| | Blindwert | 0 | 1 | 8 |
| nach 4 Wochen bei 37°C | Probe | 0 | 10 | 0 (!) |
| | Blindwert | 0 | 0 | 6 |

Die Vorteile dieses Verfahrens:
- Für die Erhaltung der bakteriologischen Qualität sind keinerlei chemische Zusätze notwendig.
- Das Wasser behält seinen natürlichen Geschmack.
- Durch die Zerschlagung der Mutterkolonien und durch die Temperaturempfindlichkeit der daraus gebildeten Tochterkolonien wird der bakteriologische Status des Trinkwassers bei Temperatureinwirkung noch verbessert.

## Patentansprüche

1. Verfahren zur Behandlung von Mikroorganismen bzw. eines Mikroorganismen enthaltenden Mediums, dadurch gekennzeichnet, daß ein in seiner elektromagnetischen Struktur durch Änderung der magnetischen Kernresonanzeigenschaften und durch Ausbildung von supermolekularen Komplexen zwischen den Molekülen in seinem Schwingungszustand geändertes Wasser oder Medium indirekt auf das die Mikroorganismen enthaltende Medium einwirkt oder diesem direkt zugesetzt wird.

2. Verfahren nach Anspruch 1, dadurch gekennzeichnet, daß diese Behandlung sein kann: Abtötung von Mikroorganismen, Keimzahlverminderung, aber auch Keimzahlerhöhung durch Umwandlung von Großkolonien in Einzelkolonien, genetische Veränderungen, Veränderungen im fermentativen Verhalten, in der Beweglichkeit und im mikroskopischen Bild, makroskopische Veränderungen im Koloniebild, in der Koloniefarbe, in der Anfärbbarkeit oder in der Geruchsbildung, Veränderungen im aeroben oder anaeroben Verhalten, in der Sporenbildung, Veränderungen bei der Bildung von Spaltprodukten sowie Veränderungen in Richtung konservierender Effekte, betreffend Mikroorganismen in Form von Bakterien, Viren und Pilzen.

3. Verwendung des Verfahrens nach Anspruch 2 zur Erhöhung der Empfindlichkeit der Mikroorganismen gegenüber Desinfektionsmitteln und/oder Temperaturerhöhung durch Umwandlung von Großkolonien von Mikroorganismen in Einzelkolonien.

4. Verwendung des Verfahrens nach Anspruch 1 oder 2 zur bakteriologischen Stabilisierung von Schwimmbadwasser.

5. Verwendung des Verfahrens nach Anspruch 1 oder 2 zur Beschleunigung der Nitrifizierung von Gülle.

6. Verwendung des Verfahrens nach Anspruch 1 oder 2 zur bakteriologischen Stabilisierung von Trinkwasser ohne geschmackliche Veränderung.

7. Verwendung nach Anspruch 6 für Trinkwasser in temperierbaren Behältern zur zahnärztlichen Verwendung als Mundspülflüssigkeit.

8. Verwendung nach Anspruch 6 für in geschlossenem Behälter, längere Zeit lagerbares Trinkwasser.

9. Verwendung des Verfahrens nach Anspruch 1 oder 2, in Schlauchsystemen zahnärztlicher Behandlungseinheiten und Amalgamabscheidern zur Vermeidung der Geruchs- und Belagsbildung, der Vermehrung von Mikroorganismen und der Verunreinigung des Abwassers durch Herauslösung von Schwermetallen aus dem Amalgam.

10. Vorrichtung des Verfahrens nach Anspruch 1 oder 2, dadurch gekennzeichnet, daß die Vorrichtung aus einem Doppelmantelgefäß besteht, wobei ein in seiner elektromagentischen Struktur und in seinem Schwingungszustand verändertes Wasser oder Medium im äußeren Mantel eingefüllt ist und damit ohne direkte Berührung auf die Mikroorganismen im Medium einwirkt, das sich im inneren Mantel befindet oder den inneren Mantel durchströmt.

11. Vorrichtung nach Anspruch 10, dadurch gekennzeichnet, daß die den äußeren und den inneren Mantel trennende Wand aus Eisen, Stahl, Edelstahl, Aluminium, Glas, Porzellan, Kupfer, Messing oder Kunststoff mit einer Wandstärke bis zum 5 mm besteht.
